# EUROPEAN PATENT SPECIFICATION

(11) **EP 3 302 405 B1**
(45) Date of publication and mention of the grant of the patent: **23.08.2023**
(21) Application number: 16723305.5
(22) Date of filing: 05.05.2016
(51) Int. Cl.: A61K 8/73, A61K 8/24, A61K 8/25, A61Q 11/00, A61C 19/06, A61K 8/02, A61K 8/81

(54) **ORAL CARE DEVICE**
MUNDPFLEGEVORRICHTUNG
DISPOSITIF DE SOIN ORAL

(30) Priority: 05.06.2015 EP 15170826
(43) Date of publication of application: 11.04.2018
(73) Proprietor: Unilever IP Holdings B.V., 3013 AL Rotterdam (NL); Unilever Global IP Limited, Wirral, Merseyside CH62 4ZD (GB)
(72) Inventor: GROVES, Brian Joseph, Wirral Merseyside CH63 3JW (GB); LIMER, Adam John, Wirral Merseyside CH63 3JW (GB); WILSON, William John, Wirral Merseyside CH63 3JW (GB)
(74) Representative: Tansley, Sally Elizabeth
(86) International application number: PCT/EP2016/060124
(87) International publication number: WO 2016/192923

(56) References cited:
- WO-A1-2008/068149
- WO-A1-2008/068149
- WO-A1-2011/109919
- WO-A2-2012/031785
- US-A1- 2006 099 550
- US-A1- 2006 292 092

## Description

### Field of the Invention

The invention relates to an oral care products for the remineralisation of teeth. The oral care products comprise a device and composition. Application of the composition using the device can lead to remineralisation, repair and in some cases whitening of the teeth.

### Background of the Invention

Calcium phosphate is the primary component of the enamel and dentin in the form of hydroxyapatite.

In the mouth, there is a natural equilibrium between hydroxyapatite being dissolved from the enamel of teeth and hydroxyapatite being formed on or in the teeth from substances occurring naturally in the saliva. This equilibrium is shifting continuously. Among other factors, it is determined by diet and physical condition.

If the equilibrium is such that hydroxyapatite is being dissolved, a cariogenic condition arises which is referred to as demineralisation. Exposure of the dental hard tissues to acid causes demineralisation, resulting in surface softening and a decrease in mineral density. Dental erosion (i.e. acid erosion or acid wear) is a surface phenomenon that involves demineralisation, and ultimately complete dissolution of the tooth surface by acids that are not of bacterial origin. Most commonly the acid will be of dietary origin.

If the equilibrium is such that hydroxyapatite is being formed, this is referred to as remineralisation. Remineralisation hardens and strengthens the teeth, thereby providing protection from and treatment for dental erosion and/or tooth wear. It also reduces the likelihood of tooth decay and improves the appearance of the teeth, in particular their whiteness. The teeth may also appear smoother and shinier as a result.

The presence of fluoride ions can enhance the natural remineralisation process and this is one of the accepted mechanisms by which fluoride toothpastes and rinses protect against caries. Fluoride is most effective during the developmental years from childhood to young adulthood.

However, the ability of fluoride to promote remineralisation can be limited by the availability of calcium and phosphate ions in saliva. Accordingly, oral products utilising calcium and phosphate ions have been designed.

Strips for enamel whitening are described in US 2006/292092 A1. WO2008/068149 A1 discloses a two component system for enamel regeneration

A single phase enamel regeneration composition is disclosed in WO 2011/109919 A1.

US 5,605,675 discloses a process for remineralisation of dental enamel by application of a two-phase composition; one phase containing a water-soluble calcium compound and one phase containing a water soluble inorganic phosphate and a water-soluble fluorine compound.

US 4,083,955 discloses a process for remineralisation of dental enamel by sequential application of two compositions, the first comprising calcium ions and second comprising phosphate ions, or vice versa.

However there remains the need for a simple and effective way to enable enamel regeneration of the teeth.

### Summary of the Invention

Accordingly the present invention relates to a delivery device for delivering a enamel regeneration system to the surfaces of teeth, as described in claim 1

### Detailed Description of the Invention

The delivery device of the invention comprises a strip of an orally acceptable flexible material. The surface of the strip is capable of being applied to a tooth surface.

Preferably the device has an elongate shape of a length sufficient that when placed against the front surface of a user's teeth it extends across a plurality of teeth, and of sufficient width that it extends at least from the gumline of the teeth to the crowns of the teeth. The elongate shape is such that it minimises the need for subsequent applications and time to cover all the users teeth.

In a further embodiment the strip is such that it can be sufficient that when placed against the front surface of a user's teeth it extends across a plurality of teeth, and of sufficient width that it extends at least from the front gumline of the teeth to the crowns of the teeth and to the gumline behind the users teeth leading to total coverage of the teeth above the gumline.

Preferably the application device is rectangular in shape.

The strip/device has the enamel regeneration system deposited upon the strip surface thereof as a layer or more preferably multiple layers.

The device comprises at least three layers; one layer comprises a non-dissolving backing film, a second layer comprises a component of the enamel regeneration system, and a third layer comprises a water soluble polymer film.

It is preferable if the non-dissolving backing layer comprises materials such as polymers, natural and synthetic wovens, non-wovens, foil, paper, rubber, and combinations thereof. The strip of material may be a single layer of material or a laminate of more than one layer. Generally, the strip of material is substantially water impermeable. The material may be any type of polymer that is compatible with tooth whitening actives and is sufficiently flexible to be shaped and applied to the tooth surface. The material may comprise a single polymer or a mixtures of polymers. Suitable polymers include, but are not limited to, polyethylene, polypropylene, ethylvinylacetate, ethylvinyl alcohol, polyesters, polyamides, fluoroplastics and combinations thereof. Preferably, the material is polyethylene. The strip of material is generally less than about 1 mm thick, preferably less than about 0.05 mm thick, and more preferably from about 0.001 to about 0.03 mm thick.

The strip has a second layer comprising components of the enamel regeneration system. It is advantageous if the component of the enamel regeneration system is a water insoluble and/or slightly soluble calcium source.

Soluble and insoluble calcium source, as used herein, refers to the solubility of the calcium source in water. Soluble means a source that dissolves in water to give a solution with a concentration of at least 0.1 moles per litre at room temperature. Insoluble means a source that dissolves in water to give a solution with a concentration of less than 0.001 moles per litre at room temperature. Slightly soluble, therefore, is defined to mean a source that dissolves in water to give a solution with a concentration of greater than 0.001 moles per litre at room temperature and less than 0.1 moles per litre at room temperature. Substantially free of, as used herein, means less than 1.5%, and preferably, less than 1.0%, and most preferably, from 0.0 to 0.75% by weight, based on total weight of the oral care composition, including all ranges subsumed therein. The calcium source suitable for use in this invention is limited only to the extent that the same may be used in an oral cavity. In a preferred embodiment, the calcium source employed is insoluble or slightly soluble in water, but most preferably, insoluble in water.

Illustrative examples of the types of calcium source that may be used include, for example, calcium phosphate (i.e., added), calcium gluconate, calcium oxide, calcium lactate, calcium carbonate, calcium hydroxide, calcium sulfate, calcium carboxymethyl cellulose, calcium alginate, calcium salts of citric acid, calcium silicate, mixtures thereof or the like. According to the invention the calcium source is calcium silicate. In a preferred embodiment, the calcium silicate used is (CaSᵢO₃) whereby the same is made commercially available under the name Microcal ET by Ineos Silicas, Ltd.

In yet another preferred embodiment, the calcium source is insoluble calcium silicate, present as the composite material calcium oxide-silica (CaO-SiO₂) as described in commonly-owned application Publication No. 2008/015117.

The ratio of calcium to silicon (Ca:Si) may be from 1:10 to 3:1. The Ca:Si ratio is preferably from 1:5 to 2:1, and more preferably, from 1:3 to 2:1, and most preferably, from about 1:2 to 2:1. The calcium silicate may comprise mono-calcium silicate, bi-calcium silicate, or tri-calcium silicate whereby ratios of calcium to silicon (Ca:Si) should be understood to be atom ratios.

The calcium source employed in this invention may be in a crystalline or amorphous state, and preferably, the same is in an amorphous state. In an often preferred embodiment, the calcium source is in a mesoporous state, i.e. the source is a material having pores with diameters from 1 nm to 50 micrometers. Mesoporous calcium silicate (MCS) is often preferred.

The MCS which may be used in this invention can be made by combining a calcium salt, a silica precursor like silicate and a structure-directing agent to yield a solid suitable for calcinating. A more detailed description of the process that may be conducted to make the MCS suitable for use in this invention is described in the aforementioned commonly-owned application, Publication No. WO 2008/015117.

The amount of calcium source in the composition present as the second layer of this invention is typically from 0.1 to 50%, and preferably, from 1 to 30%, and most preferably, from 5 to 20% by weight of the oral care composition based on total weight of the oral care composition and including all ranges subsumed therein.

The second layer is non-aqueous, that is that the composition comprises less than 1 wt% of the total composition of the second layer of water, preferably less than 0.5 wt% of water.

It is highly preferable if the second layer is sandwiched between the first backing layer and a third layer.

The device comprises a third layer. The third layer comprises a water soluble polymer or polymers. Suitable water soluble polymers may be natural or synthetic. Suitable natural hydrocolloids and water soluble polymers include cellulosic material, a polysaccharide, a gum, a protein, a starch or a glucan. Examples include but are not limited to carboxymethyl cellulose, hydroxyl methyl cellulose, hydroxyethyl cellulose, hydroxypropyl cellulose, hydroxypropylmethyl cellulose, gum Arabic, xanthan gum, karaya gum, tragacanth, acacia, carrageenan, guar gum, locust bean gum, pectin, alginates, polydextrose, dextrin, dextran, amylose, chitin, chitosan, levan, elsinan, collagen, gelatin, zein, gluten, soy protein isolate, whey protein isolate, casein and pullan. Suitable water soluble synthetic polymers include but are not limited to poly(vinyl pyrrolidone), poly(vinyl alcohol), poly(acrylic acid), polyacrylates, Methylmethacrylate copolymer, carboxyvinyl polymer, polyethylene oxide and polyethylene glycol. The water soluble polymer layer is preferably a mixture of methylcellulose and hydroxypropyl methylcellulose.

The third layer further comprises a phosphate source. The phosphate source that may be used in this invention is limited only to the extent that the same may be used in a composition suitable for use in an oral cavity. Illustrative examples of the types of phosphate source suitable for use in this invention include monosodium phosphate, sodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate, mixtures thereof or the like. The phosphate source is preferably one which is water soluble.

Typically, the phosphate source makes up from 0.5 to 15%, and preferably, from 2 to 12%, and most preferably, from 4 to 9% by weight of the composition used in the third layer, based on total weight of the composition of the third layer and including all ranges subsumed therein. In a preferred embodiment, the phosphate source used is one which results in an oral care composition having a pH from 5.5 to 8, preferably from 6 to 7.5, and most preferably, about neutral. In a most preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

It is preferable if the composition of the third layer comprises an aqueous base, that is that that the composition comprises greater than 50 wt% of the third composition of water, more preferably greater than 70 wt%.

The advantage of having a water soluble layer and a non-water soluble layer is that in the mouth the water soluble layer dissolves in the saliva and the ingredients enclosed therein (the phosphate) can react with the ingredients in the second layer (silicate) to aid the in-situ regeneration of enamel on the teeth. Thus the system provides an enamel regeneration system that can be used with the minimum of water, can be easily stored and is stable on storage.

The composition of the invention comprise titanium dioxide. The titanium dioxide is present in the second layer. A preferred form of titanium dioxide is calcium silicate coated titanium dioxide. Examples of preferred forms of calcium silicate coated titanium dioxide are disclosed in WO2012/031786 and WO2012/03178.

It is preferable if the thickness of the first layer is less than about 1 mm thick, preferably less than about 0.05 mm thick, and more preferably from about 0.001 to about 0.03 mm thick, second layer is less than about 1 mm thick, preferably less than about 0.5 mm thick, and more preferably from about 0.001 to about 0.3 mm thick and third layer (if present)is less than about 1 mm thick, preferably less than about 0.5 mm thick, and more preferably from about 0.001 to about 0.3 mm thick.

The oral care composition forming the layers described herein may comprise ingredients which are common in the art, such as:
▪ antimicrobial agents, e.g. Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds such as 2,2' methylenebis-(4-chloro-6-bromophenol);
▪ anti-inflammatory agents such as ibuprofen, flurbiprofen, aspirin, indomethacin, etc.;
▪ anti-caries agents such as sodium trimetaphosphate and casein;
▪ plaque buffers such as urea, calcium lactate, calcium glycerophosphate and polyacrylates;
▪ vitamins such as Vitamins A, C and E;
▪ plant extracts;
▪ desensitizing agents, e.g. potassium citrate, potassium chloride, potassium tartrate, potassium bicarbonate, potassium oxalate, and potassium nitrate;
▪ anti-calculus agents, e.g. alkali-metal pyrophosphates, hypophosphite-containing polymers, organic phosphonates and phosphocitrates, etc.;
▪ biomolecules, e.g. bacteriocins, antibodies, enzymes, etc ;
▪ flavors, e.g., peppermint and spearmint oils;
▪ proteinaceous materials such as collagen;
▪ preservatives;
▪ opacifying agents;
▪ coloring agents like FD&C blue, yellow and/or red dyes/colorants;
▪ pH-adjusting agents;
▪ sweetening agents;
▪ surfactants, such as anionic, cationic and zwitterionic or amphoteric surfactants (e.g., sodium lauryl sulfate, sodium dodecylbenzene sulfonate);
▪ particulate abrasive materials such as abrasive silicas, aluminas, calcium carbonates, zirconium silicate, polymethylmethacrylate, dicalciumphosphates, calcium pyrophosphates, hydroxyapatites, trimetaphosphates, insoluble hexametaphosphates as well as agglomerated particulate abrasive materials;
▪ fluoride sources like sodium fluoride, stannous fluoride, sodium monofluorophosphate, zinc ammonium fluoride, tin ammonium fluoride, calcium fluoride, cobalt ammonium fluoride or mixtures thereof;
▪ polymeric compounds which can enhance the delivery of active ingredients such as antimicrobial agents can also be included. Examples of such polymers are copolymers of polyvinylmethylether with maleic anhydride and other similar delivery enhancing polymers, e.g., those described in DE-A03,942,643; buffers and salts to buffer the pH and ionic strength of the oral care compositions; and
▪ other optional ingredients that may be included are, e.g., bleaching agents such as peroxy compound, e.g., potassium peroxydiphosphate, effervescing systems such as sodium bicarbonate/citric acid systems, color change systems, and the like.

Such ingredients common in the art typically and collectively make-up less than 20% by weight of the oral care composition, and preferably, from 0.0 to 15% by weight, and most preferably, from about 0.01 to about 12% by weight of the oral care composition, including all ranges subsumed therein.

Suitable carrier humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. The carrier humectants should, in any case, be substantially free of water, and preferably, anhydrous. The same, for example, can be used in solid form, whereby glycerin is the preferred carrier humectant. Such carriers are particularly suitable in compositions used in the second layer.

The carrier humectant is used to take the balance of the compositions up to 100%, and the same may be present in the range of from 10 to 90% by weight of the oral care composition. Preferably, the carrier humectant makes up from 25 to 80%, and most preferably, from 45 to 70% by weight of the oral care composition, based on total weight of the oral care composition and including all ranges subsumed therein.

The composition used in the layers of the invention are prepared by conventional methods of making oral care formulations. Such methods include mixing the ingredients under moderate shear and atmospheric pressure.

### Mode of Use

The invention a provides a method of remineralising teeth, in which the oral care device as described above is applied to the surface of the teeth for sustained contact.

In the context of the present invention sustained contact means the product is preferably left on the teeth for 1 to 60 minutes, more preferably, for 5 to 45 minutes, most preferably from 10 to 30 minutes before being removed.

Preferably the application of the oral care product of the invention is carried out once daily for a period of several consecutive days, in addition to a regular regime of tooth brushing (preferably at least twice daily).

Typically, use (for a period of about two weeks to one month) of the device with the oral care composition of the present invention will result in a new hydroxyapatite layer on teeth that is from 0.5 to 20 microns, and preferably, from 0.75 to 5 microns, including all ranges subsumed therein.

### Detailed Description of non-limiting embodiments

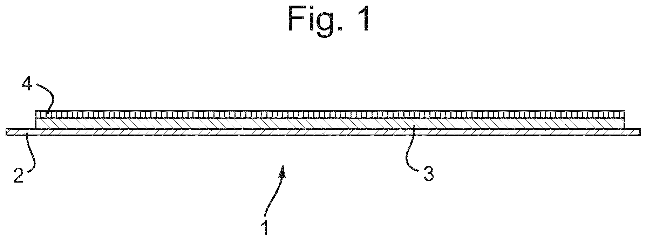

The invention, however, may be best understood by reference to the following description of some embodiments of the invention taken in conjunction with the accompanying drawing figure in which figure 1 is a cross sectional view of the strip.

Referring to figure 1, a device (1) of the invention is shown in section. The device comprises a strip (2) of insoluble backing material, this can be considered the bottom layer. Coated onto the insoluble backing material (2) is a second layer (3) comprising calcium silicate and titanium dioxide composite, a third layer (4) is present as the top layer in contact with the second layer. The third layer comprises a phosphate salt and water soluble polymer. In a preferred embodiment the phosphate salt comprises trisodium phosphate and monosodium dihydrogen phosphate and the polymer is a mixture of methylcellulose and hydroxypropyl methylcellulose.

The invention will now be illustrated by the following non-limiting Examples:

### Examples

A simulated oral fluid was prepared by adding 1.9L of water to a glass beaker. The following materials were added one by one with continuous stirring, allowing sufficient time for each chemical to fully dissolve before adding the next. Sodium chloride 16.07g, sodium hydrogen carbonate 0.7g, potassium chloride 0.448g, potassium hydrogen phosphate 2.56g, magnesium chloride hexahydrate 0.622g, 1M hydrochloric acid 40ml, calcium chloride 0.1998g and sodium sulfate 0.1434g. The pH was adjusted to 7.0 using saturated TRIS buffer and the total volume made up to 2L using a volumetric flask.

### Formulation and processing: Polymer film

| **Ingredient** | **%w/w** |
|---|---|
| Glycerol | 1.0 |
| Monosodium phosphate | 3.2 |
| Trisodium phosphate | 3.8 |
| Hydroxypropyl cellulose | 4.0 |
| Methyl cellulose | 4.0 |
| Water | 84 |
| Total | 100 |

The water and glycerol was weighed into a beaker. The polymers added slowly while under shear using a Silverson LR4 bench top mixer. When the polymer is fully dispersed (no lumps visible) the phosphates are added and mixed to disperse.

NB Air can become entrapped in the solution if required this can be removed using a desiccator.

To cast the film a quantity of the solution is poured on to a glass tile and using a pull down gauge the correct film height is achieved (micrometers on the gauge set at 15). The glass tiles are then placed in an oven at 50-60DegC over night to remove the water.

The following day the film can be removed from the tile and cut to the appropriate size.

### Formulation and processing: Tio2/Cs Slurry

| **Ingredient** | **%w/w** |
|---|---|
| Glycerol | 75 |
| Calcium Silicate | 10 |
| TiO2/CS | 15 |
| Total | 100 |

Glycerol was weighed into a suitable beaker then the two powders were added slowly while under shear using a Silverson LR4 bench top mixer. The slurry was mixed to remove any visible lumps.

The multilayer film was produced as follows.

Stage 1 a piece of plastic film was cut to the appropriate size.

Stage 2 using a four point balance 0.04g of Slurry was dispensed onto the film.

Stage 3 a rectangle shaped piece of the MSP/TSP polymer film (weighing approx. 0.04g) was cut and placed on top of the slurry and pressed evenly to dispersed the slurry equally under the film.

To a strip sample was added 0.5ml deionized water via auto-pipette and immediately three 6x6mm enamel block samples were placed on the surface. The samples were left in contact with the strip for 30 minutes with gentle agitation at 5 and 15 minutes. This procedure was completed twice, so n=6 enamel blocks were tested. After 30 minutes the bovine blocks were rinsed in 40 ml water and mixed gently for 1 minute. Samples were then incubated in simulated oral fluid for 5 hours. The application process was repeated 5 times in total. Colour was measured via chromameter at baseline and after strip application and incubation. L*a*b* colour parameters were converted to WIO whiteness indices to allow comparison between samples. Colour change is expressed as ΔWIO = WIO(slurry application)-WIO(baseline).

| | **1 Application** | **3 Applications** | **5 Applications** |
|---|---|---|---|
| Whitening Change | 3.02 ± 0.69 | 4.35 ± 1.00 | 6.36 ± 0.74 |

## Claims

1. A delivery device for delivering a enamel regeneration system to the surfaces of teeth, the delivery device **characterised in that** it comprises plurality of layers comprising:
i) a first layer comprising a strip of an orally acceptable flexible, non-aqueous dissolving material capable of being applied to a tooth surface;
ii) a second non-aqueous layer comprising a component of the enamel regeneration system comprising calcium silicate and tooth whitening substance comprising titanium dioxide and less than 1 wt% of the total composition of water
iii) a third layer comprising a water soluble polymer film and a phosphate source.

2. A device according to any preceding claim in which the phosphate source comprises trisodium phosphate, sodium dihydrogen phosphate or mixtures thereof.

3. A device according to any preceding claim in which the water soluble polymer of the third layer comprises a cellulose.

4. A device according to any preceding claims having an elongate shape of a length sufficient that when placed against the front surface of a user's teeth it extends across a plurality of teeth, and of sufficient width that it extends at least from the gumline of the teeth to the crowns of the teeth.

5. A device according to any preceding claim being substantially rectangular.

## Patentansprüche

1. Abgabevorrichtung zur Abgabe eines Systems zur Regeneration des Zahnschmelzes auf Zahnoberflächen, wobei die Abgabevorrichtung **dadurch gekennzeichnet ist, dass** sie eine Vielzahl von Schichten umfasst, umfassend:
i) eine erste Schicht, die einen Streifen aus einem oral verträglichen, flexiblen, nicht-wässrigen, auflösbaren Material umfasst, um auf eine Zahnoberfläche aufgetragen zu werden;
ii) eine zweite nicht-wässrige Schicht, umfassend eine Komponente des Systems zur Regeneration des Zahnschmelzes, umfassend Calciumsilikat und Zahnaufhellungssubstanz, umfassend Titandioxid und weniger als 1 Gew.-% der gesamten Zusammensetzung an Wasser,
iii) eine dritte Schicht, umfassend einen wasserlöslichen Polymerfilm und eine Phosphatquelle.

2. Vorrichtung nach einem vorhergehenden Anspruch, in der die Phosphatquelle Trinatriumphosphat, Natriumdihydrogenphosphat oder Mischungen davon umfasst.

3. Vorrichtung nach einem vorhergehenden Anspruch, in der das wasserlösliche Polymer der dritten Schicht Cellulose umfasst.

4. Vorrichtung nach einem der vorhergehenden Ansprüche, die eine längliche Form einer Länge aufweist, die ausreicht, dass dann, wenn sie auf die Vorderfläche der Zähne eines Benutzers angelegt wird, sich über mehrere Zähne erstreckt und ausreichend breit ist, dass sie sich zumindest vom Zahnfleischrand der Zähne bis zu den Zahnkronen erstreckt.

5. Vorrichtung nach einem vorhergehenden Anspruch, die im Wesentlichen rechteckig ist.

## Revendications

1. Dispositif de distribution pour distribuer un système de régénération d'émail sur les surfaces de dents, le dispositif de distribution **caractérisé en ce qu'**il comprend plusieurs couches comprenant :
i) une première couche comprenant une bande d'une matière de dissolution non aqueuse, flexible oralement acceptable pouvant être appliquée sur une surface de dent ;
ii) une seconde couche non-aqueuse comprenant un constituant du système de régénération d'émail comprenant du silicate de calcium et une substance de blanchiment des dents comprenant du dioxyde de titane et moins de 1 % en masse de la composition totale d'eau
iii) une troisième couche comprenant un film polymère soluble dans l'eau et une source de phosphate.

2. Dispositif selon l'une quelconque des revendications précédentes, dans lequel la source de phosphate comprend du phosphate de trisodium, dihydrogénophosphate de sodium ou des mélanges de ceux-ci.

3. Dispositif selon l'une quelconque des revendications précédentes, dans lequel le polymère soluble dans l'eau de la troisième couche comprend une cellulose.

4. Dispositif selon l'une quelconque des revendications précédentes ayant une forme allongée d'une longueur suffisante de sorte que lorsque il est placé contre la surface avant d'une dent d'utilisateur il s'étend à travers plusieurs dents, et de largeur suffisante de sorte qu'il s'étend au moins à partir de la gencive des dents jusqu'aux couronnes des dents.

5. Dispositif selon l'une quelconque des revendications précédentes étant substantiellement rectangulaire.
